Europäisches Patentamt

European Patent Office

Office européen des brevets

(19)

(11) Veröffentlichungsnummer: **0 063 341**
**B1**

(12) EUROPÄISCHE PATENTSCHRIFT

(45) Veröffentlichungstag der Patentschrift:
21.11.84

(51) Int. Cl.³: **A 61 M 5/30**

(21) Anmeldenummer: **82103079.8**

(22) Anmeldetag: **09.04.82**

(54) Kolbenpumpe für nadellose Injektionsgeräte.

(30) Priorität: **16.04.81 DE 3115375**

(43) Veröffentlichungstag der Anmeldung:
**27.10.82 Patentblatt 82/43**

(45) Bekanntmachung des Hinweises auf die Patenterteilung:
**21.11.84 Patentblatt 84/47**

(84) Benannte Vertragsstaaten:
**CH DE FR GB LI**

(56) Entgegenhaltungen:
**DE - A - 1 491 833**
**US - A - 3 515 130**
**US - A - 4 184 256**

(73) Patentinhaber: **HOECHST AKTIENGESELLSCHAFT,
Postfach 80 03 20, D-6230 Frankfurt am Main 80 (DE)**

(72) Erfinder: **Dettbarn, Hans-Jürgen, Rehbocks Ecke 4,
D-3550 Marburg/Lahn (DE)**
Erfinder: **Zimmermann, Josef, Auf der Krautweide 11,
D-6231 Sulzbach (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

BUNDESDRUCKEREI BERLIN

## Beschreibung

Die Erfindung betrifft eine Kolbenpumpe für nadellose Injektionsgeräte, bei der in der Pumpenkammer ein Ventilkörper mit Auslaßkanal und Austrittsventil für das zu injizierende Medium angeordnet ist.

Kolbenpumpen der genannten Art sind aus der deutschen Auslegeschrift 1 213 958 und der US-Patentschrift 3 526 225 bekannt. Im Pumpenzylinder der dort gezeigten Kolbenpumpen ist ein Ventilkörper angeordnet, der mit einem Auslaßkanal für dem Impfstoff versehen ist. Im Auslaßkanal ist ein federbelastetes Kugelventil als Rückschlagventil angeordnet. Nachteilig ist die getrennte Anordnung von Einlaßkanal und Auslaßkanal für das Reinigen der Impfstoffpumpe und der relativ große Platzbedarf, der für solche Anordnungen erforderlich ist. Es ist ferner von Nachteil, daß durch die getrennte Anordnung von Einlaßkanal und Auslaßkanal ein großes Restvolumen an Impfstoff in der Pumpe selbst nach erfolgtem Schuß zurückbleibt.

Hier will die Erfindung Abhilfe schaffen. Die Erfindung, wie sie in den Ansprüchen gekennzeichnet ist, löst die Aufgabe dadurch, daß der Ventilkörper einen Einlaßkanal zum Zuführen des zu injizierenden Mediums in die Pumpenkammer aufweist und in einer Erweiterung des Einlaufkanals eine Rückschlagklappe angeordnet ist und daß der Auslaßkanal mit einer Ringnut im Ventilkörper in Verbindung steht, in der sich ein Dichtring befindet, der im Zusammenwirken mit der Ringnut die Funktion des Austrittsventils mit Rückschlagsicherung erfüllt.

Die Rückschlagklappe ist zweckmäßig mit einer Sicherung gegen Herausfallen aus der Erweiterung und einem Führungszapfen versehen, der in den Einlaßkanal hineinragt. Zur Erleichterung der Justierung des Ventilkörpers in der Pumpenkammer kann dem Einlaßkanal eine Einlaufringnut zugeordnet sein, die zwischen zwei Ringnuten zur Aufnahme von Dichtungen angeordnet ist.

Die durch die Erfindung erreichten Vorteile sind im wesentlichen darin zu sehen, daß durch die Anordnung von Einlaßkanal und Auslaßkanal mit ihren jeweiligen Ventilen in einem Ventilkörper, der mit einer als Überwurfmutter ausgebildeten Düse in der Pumpenkammer gehalten wird, die Kolbenpumpe einfach und effektiv zu reinigen ist. Nach Herausziehen des Ventilkörpers aus der Pumpenkammer sind alle Kanäle und Ventile direkt zugänglich. Ein weiterer Vorteil ist, daß alle Wege für das zu injizierende Medium kurz gehalten werden können, so daß nach erfolgtem Schuß nur noch eine geringe Restmenge im Pumpensystem verbleibt.

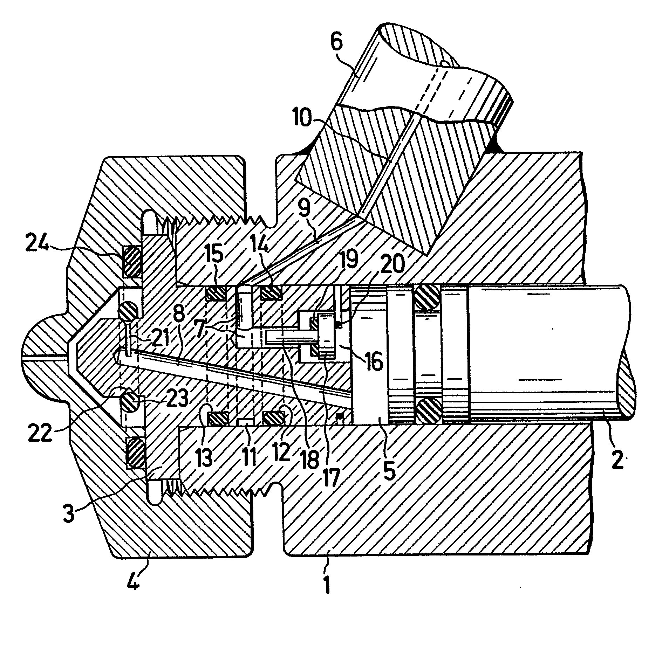

Im folgenden wird die Erfindung anhand von einer lediglich einen Ausführungsweg darstellenden Zeichnung näher erläutert. Es zeigt die Figur einen Teil der Kolbenpumpe geschnitten.

Die Kolbenpumpe besteht aus dem Pumpengehäuse (1) mit Pumpenkammer (5), dem Pumpenkolben (2), dem Ventilkörper (3) und der Düse (4). Das Pumpengehäuse weist ferner eine Einrichtung (6) zur Aufnahme eines Behälters für das zu injizierende Medium auf.

Einlaßkanal (7) und Auslaßkanal (8) für das zu injizierende Medium sind in einem gemeinsamen Ventilkörper (3) angeordnet. Der Ventilkörper (3) ist in der Pumpenkammer (5) des Pumpengehäuses (1) zentriert. Das Pumpengehäuse (1) ist mit einer Bohrung (9) versehen, die den Kanal (10) der Einrichtung (6) mit dem Einlaßkanal (7) verbindet. Um unnötiges Justieren des Ventilkörpers (3) in der Pumpenkammer (5) zu vermeiden, kann dem Einlaßkanal eine Einlaufringnut (11) zugeordnet sein, wodurch unabhängig von der Stellung des Ventilkörpers in der Pumpenkammer der freie Weg zwischen Bohrung (9) und Einlaßkanal (7) sichergestellt ist. Beiderseits der Einlaufringnut (11) sind Ringnuten (12) und (13) zur Aufnahme von Dichtringen (14) und (15) vorgesehen. In einer Erweiterung des Einlaßkanals (7) ist eine Rückschlagklappe (16) bestehend aus Ventilteller (17), Führungszapfen (18) und Dichtung (19) angeordnet. Die Rückschlagklappe (16) wird durch eine Sicherung (20) im Einlaßkanal (7) gehalten. Der Auslaßkanal (8) mündet direkt in eine Ringnut (22) oder steht durch eine oder mehrere Bohrungen oder einen Schlitz (21) mit derselben in Verbindung. Die Ringnut (22) ist vorzugsweise V-förmig ausgebildet. In ihr ist ein Dichtring (23) angeordnet, der im Zusammenwirken mit der Ringnut (22) die Funktion eines Austrittsventils mit Rückschlagsicherung erfüllt. Bei Überdruck im Pumpenraum schließt Rückschlagklappe (16) und das zu injizierende Medium gelangt über Auslaßkanal (8) und Ringnut (22) zur Düse (4). Bei Unterdruck im Pumpenraum schließt Dichtring (23) die Ringnut (22) und damit den Auslaßkanal; Rückschlagklappe (16) öffnet; zu injizierendes Medium kann über Einlaßkanal (7) in die Pumpenkammer (5) gelangen. Ein Ansaugen von Flüssigkeit und/oder Luft durch die Düse (4) ist nicht möglich. (24) deutet eine Dichtung in Düse (4) an.

## Patentansprüche

1. Kolbenpumpe für nadellose Injektionsgeräte, bei der in der Pumpenkammer (5) ein Ventilkörper (3) mit Auslaßkanal (8) und Austrittsventil für das zu injizierende Medium angeordnet ist, dadurch gekennzeichnet, daß der Ventilkörper (3) einen Einlaßkanal (7) zum Zuführen des zu injizierenden Mediums in die Pumpenkammer (5) aufweist und in einer Erweiterung des Einlaßkanals (7) eine Rückschlagklappe (16) angeordnet ist und daß der Auslaßkanal (8) mit einer Ringnut (22) im Ventilkörper (3) in Verbindung steht, in der sich ein Dichtring (23) befindet, der im Zusammenwirken mit der Ringnut (22) die Funktion des Austrittsventils mit Rückschlagsicherung erfüllt.

2. Kolbenpumpe nach Anspruch 1, dadurch gekennzeichnet, daß die Rückschlagklappe (16) mit einer Sicherung (20) gegen Herausfallen aus der Erweiterung und einem Führungszapfen (18) versehen ist, der in den Einlaßkanal (7) hineinragt.

3. Kolbenpumpe nach Anspruch 1 oder 2, dadurch gekennzeichnet, daß dem Einlaßkanal (7) eine Einlaufringnut (11) zugeordnet ist, und die Einlaufringnut (11) zwischen zwei Ringnuten (12, 13) zum Aufnehmen von Dichtungen (14, 15) angeordnet ist.

## Claims

1. A piston pump for needle-less injection instruments, in which a valve body (3) an outlet channel (8) and outlet valve for the medium to be injected is located in the pump chamber (5), wherein the valve body (3) has an inlet channel (7) for feeding into the pump chamber (5) the medium to be injected, and a non-return flap (16) is located in a widened portion of the inlet channel (7), and the outlet channel (8) is connected to an annular groove (22) in the valve body (3) in which a sealing ring (23) is located which acts in cooperation with the annular groove (22) as a non-return outlet valve.

2. A piston pump as claimed in claim 1, wherein the non-return flap (16) is provided with a safeguard (20) against falling out from the widened portion and with a guide pin (18) which projects into the inlet channel (7).

3. A piston pump as claimed in claim 1 or 2, wherein an annular inflow groove (11) is assigned to the inlet channel (7), and the annular inflow groove (11) is located between two annular grooves (12, 13) for receiving gaskets (14, 15).

## Revendications

1. Pompe à piston pour appareils d'injection sans aiguille, dans laquelle un corps de soupape (3) muni d'un conduit de sortie (8) et d'une soupape de sortie pour le milieu à injecter est agencé dans la chambre (5) de la pompe, caractérisée en ce que le corps de soupape (3) présente un conduit d'entrée (7) pour l'introduction du milieu à injecter dans la chambre (5) de la pompe, et qu'un clapet anti-retour (16) est agencé dans un élargissement du conduit d'entrée (7), et en ce que le conduit de sortie (8) est en communication avec une gorge annulaire (22) ménagée dans le corps (3) de la soupape et dans laquelle se trouve une bague d'étanchéité (23) qui, en coopération avec la gorge annulaire (22), assure la fonction de la soupape de sortie avec sécurité anti-retour.

2. Pompe à piston selon la revendication 1, caractérisée en ce que le clapet anti-retour (16) est muni d'une sécurité (20) qui l'empêche de s'échapper de l'élargissement et d'un téton de guidage (18) qui fait saillie dans le conduit d'entrée (7).

3. Pompe à piston selon la revendication 1 ou 2, caractérisée en ce qu'au conduit d'entrée (7) est associée une gorge annulaire d'entrée (11) et en ce que la gorge annulaire d'entrée (11) est disposée entre deux gorges annulaires (12, 13) destinées à recevoir des garnitures d'étanchéité (14, 15).